(19) Europäisches Patentamt
European Patent Office
Office européen des brevets

(11) Publication number: 0 403 882 A2

(12) **EUROPEAN PATENT APPLICATION**

(21) Application number: 90110742.5

(51) Int. Cl.[5]: C07D 451/02, A61K 31/46

(22) Date of filing: 07.06.90

(30) Priority: 23.06.89 GB 8914493
30.04.90 GB 9009643

(43) Date of publication of application:
27.12.90 Bulletin 90/52

(84) Designated Contracting States:
AT BE CH DE DK ES FR GB GR IT LI LU NL SE

(71) Applicant: FUJISAWA PHARMACEUTICAL CO., LTD.
4-7, Doshomachi 3-chome Chuo-ku
Osaka-shi Osaka 541(JP)

(72) Inventor: Kato, Masayuki
16-12, Gorgyo-oeyamacho 6-chome,
Nishikyo-ku
Kyoto-shi, Kyoto 610-11(JP)
Inventor: Ito, Kiyotaka
2-10, Midorigaoka 2-chome
Ikeda-shi, Osaka 563(JP)
Inventor: Takasugi, Hisashi
14-33, Hamaguchinishi 1-chome, Suminoe-ku
Osaka-shi, Osaka 559(JP)

(74) Representative: Türk, Gille, Hrabal
Brucknerstrasse 20
D-4000 Düsseldorf 13(DE)

(54) **Indole derivatives and processes for preparation thereof.**

(57) A compound of the formula :

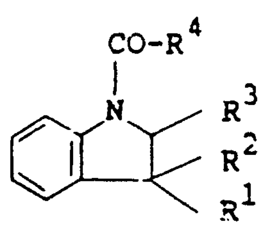

wherein
$R^1$ is lower alkyl and
$R^2$ and $R^3$ are each hydrogen, lower alkyl or linked together to form a bond; or
$R^1$ and $R^2$ are linked together to form lower alkylene and
$R^3$ is hydrogen, and
$R^4$ is azabicyclo($C_5$-$C_{12}$)alkyl(lower)alkyl or its N-oxide, each of which may be substituted by lower alkyl, provided that $R^4$ is N-oxide of azabicyclo($C_5$-$C_{12}$(lower)alkyl, which may be substituted by lower alkyl when $R^1$ is lower alkyl and $R^2$ and $R^3$ are each hydrogen or lower alkyl,
and pharmaceutically acceptable salt thereof,
processes for their preparation and pharmaceutical compositions comprising them as active ingredient in association with a pharmaceutically acceptable carrier or excipient.

EP 0 403 882 A2

# INDOLE DERIVATIVES AND PROCESSES FOR PREPARATION THEREOF

The present invention relates to novel indole derivatives and a pharmaceutically acceptable salt thereof. More particularly, it relates to novel indole derivatives and a pharmaceutically acceptable salt thereof which have pharmacological activities such as 5-hydroxytryptamine (5-HT) antagonism and the like, to processes for preparation thereof, to a pharmaceutical composition comprising the same and to a use of the same as a medicament.

Accordingly, one object of the present invention is to provide novel indole derivatives and a pharmaceutically acceptable salt thereof, which are useful as a potent and selective antagonist of 5-HT receptor.

Another object of the present invention is to provide processes for preparation of said indole derivatives or a salt thereof.

A further object of the present invention is to provide a pharmaceutical composition comprising, as an active ingredient, said indole derivatives or a pharmaceutically acceptable salt thereof.

Still further object of the present invention is to provide a use of said indole derivatives or a pharmaceutically acceptable salt thereof as a 5-HT antagonist useful for treating or preventing central nervous system (CNS) disorders such as psychosis (e.g. schizophrenia, mania, etc.), anxiety, and depression; pains or aches such as headaches (e.g. migraine, cluster headaches, vascular headaches, etc.) and neuralgia (e.g. trigeminal neuralgia, etc.); gastrointestinal disorders such as symptoms of gastrointestinal dysfunction such as occur with, for example, dyspepsia, peptic ulcer, reflux oesophagitis and flatulence, and irritable bowel syndrome (IBS); nausea or vomiting, each of which may be associated with cancer therapy; motion sickness; and the like in human being or animals, particularly nausea and vomiting.

The indole derivatives of the present invention are novel and can be represented by the formula (I) :

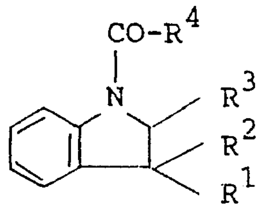

(I)

wherein

$R^1$ is lower alkyl and

$R^2$ and $R^3$ are each hydrogen, lower alkyl or linked together to form a bond; or

$R^1$ and $R^2$ are linked together to form lower alkylene and

$R^3$ is hydrogen, and

$R^4$ is azabicyclo($C_5$-$C_{12}$)alkyl(lower)alkyl or its N-oxide, each of which may be substituted by lower alkyl,

Provided that $R^4$ is N-oxide of azabicyclo($C_5$-$C_{12}$)-alkyl(lower)alkyl, which may be substituted by lower alkyl when $R^1$ is lower alkyl and $R^2$ and $R^3$ are each hydrogen or lower alkyl.

With regard to the compound (I) of the present invention, it is to be noted that there may be one or more optically or geometrically isomeric pairs due to the presence of one or more asymmetric carbon atom(s) or double bond and these isomers or a mixture thereof are included within a scope of the compound (I) of the present invention.

Particularly, the compound (I) of the present invention may adopt an endo or exo configuration, and in such a case, the endo configuration is preferred.

The optical and geometrical isomers may be separated one from the other by the usual manners.

According to the present invention, the object compound (I) can be prepared by the following processes :

<u>Process 1</u> :

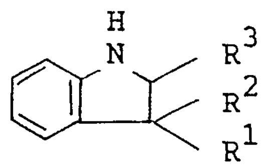

+

$HOOC-R^4$

(II)

or its reactive derivative at the imino group or a salt thereof

(III)

or its reactive derivative at the carboxy group or a salt thereof

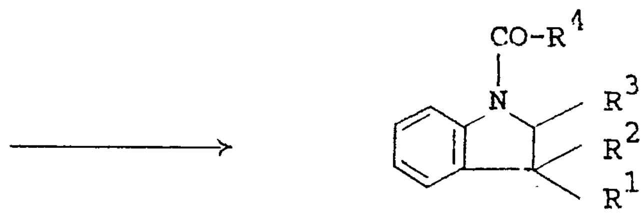

(I)

or a salt thereof

Process 2 :

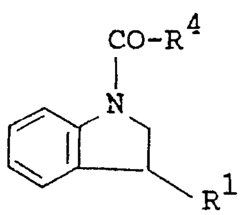

(Ia)

or a salt thereof

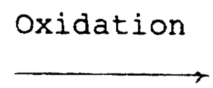


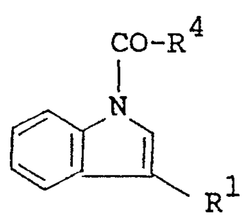

(Ib)

or a salt thereof

Process 3 :

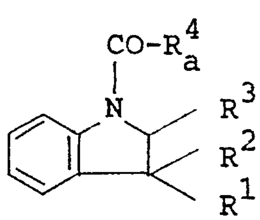

(Ic)

or a salt thereof

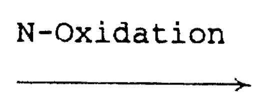

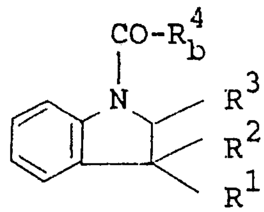

(Id)

or a salt thereof

wherein

$R^1$, $R^2$, $R^3$ and $R^4$ are each as defined above,

$R^4_a$ is azabicyclo($C_5$-$C_{12}$)alkyl(lower)alkyl which may be substituted by lower alkyl and

$R^4_b$ is N-oxide of azabicyclo($C_5$-$C_{12}$)alkyl(lower)alkyl, which may be substituted by lower alkyl.

Suitable salt of the compounds (I), (Ia), (Ib), (Ic), (Id), (II), and (III) are conventional non-toxic, pharmaceutically acceptable salt and may include a salt with an acid addition salt such as an inorganic acid addition salt (e.g. hydrochloride, hydrobromide, sulfate, phosphate, etc.); an organic carboxylic or sulfonic acid addition salt (e.g. formate, acetate, trifluoroacetate, maleate, tartrate, methanesulfonate, benzenesulfonate, p-toluenesulfonate, etc.).

In the above and subsequent descriptions of the present specification, suitable examples and illustrations of the various definitions which the present invention include within the scope thereof are explained in detail as follows.

The term "lower" is intended to mean 1 to 6 carbon atoms, preferably 1 to 4 carbon atoms, unless otherwise indicated.

Suitable "lower alkyl" may include straight or branched one, having 1 to 6 carbon atom(s), such as methyl, ethyl, propyl, isopropyl, butyl, t-butyl, pentyl, hexyl, and the like, in which the most preferred one is methyl.

Suitable "lower alkylene" is one having 1 to 6 carbon atom(s) and may include methylene, ethylene, trimethylene, propylene, tetramethylene, methyltrimethylene, dimethylethylene, hexamethylene, and the like, in which the preferred one is ethylene or tetramethylene.

Suitable "azabicyclo($C_5$-$C_{12}$)alkyl(lower)alkyl or its N-oxide" may include azabicyclo[3.2.1]octyl(lower)-alkyl or its N-oxide, azabicyclo[2.2.2]octyl(lower)alkyl or its N-oxide, azabicyclo[3.3.1]nonyl(lower)alkyl or its N-oxide, and the like, which may be substituted by lower alkyl as stated above (e.g. methyl, ethyl, etc.), preferably azabicyclo($C_7$-$C_{10}$)alkyl(lower)alkyl or its N-oxide, more preferably azabicyclo($C_8$-$C_9$)alkyl(lower)-alkyl or its N-oxide and the most preferably (8-methyl-8-azabicyclo[3.2.1]octyl)methyl or its N-oxide.

The processes 1 to 3 for preparing the object compound (I) of the present invention are explained in detail in the following.

Process 1 :

The compound (I) or a salt thereof can be prepared by reacting a compound (II) or its reactive derivative at the imino group or a salt thereof with a compound (III) or its reactive derivative at the carboxy group or a salt thereof.

Suitable reactive derivative at the imino group of the compound (II) may include a silyl derivative formed by the reaction of the compound (II) with a silyl compound such as bis(trimethylsilyl)acetamide, mono(trimethylsilyl)acetamide, bis(trimethylsilyl)urea or the like; a derivative formed by reaction of the compound (II) with phosphorus trichloride or phosgene, and the like.

Suitable reactive derivative at the carboxy group of the compound (III) may include an acid halide, an acid anhydride, an activated amide, an activated ester, and the like. Suitable examples of the reactive derivatives may be an acid chloride; an acid azide; a mixed acid anhydride with an acid such as substituted phosphoric acid [e.g. dialkylphosphoric acid, phenylphosphoric acid, diphenylphosphoric acid, dibenzylphosphoric acid, halogenated phosphoric acid, etc.], dialkylphosphorous acid, sulfurous acid, thiosulfuric

acid, sulfuric acid, sulfonic acid [e.g. methanesulfonic acid, etc.], aliphatic carboxylic acid [e.g. acetic acid, propionic acid, butyric acid, isobutyric acid, pivalic acid, pentanoic acid, isopentanoic acid, 2-ethylbutyric acid, trichloroacetic acid, etc.] or aromatic carboxylic acid [e.g. benzoic acid, etc.]; a symmetrical acid anhydride; an activated amide with imidazole, 4-substituted imidazole, dimethylpyrazole, triazole or tetrazole; or an activated ester [e.g. cyanomethyl ester, methoxymethyl ester, dimethyliminomethyl $[(CH_3)_2\overset{+}{N}=CH-]$ ester, vinyl ester, propargyl ester, p-nitrophenyl ester, 2,4-dinitrophenyl ester, trichlorophenyl ester, pentachlorophenyl ester, mesylphenyl ester, phenylazophenyl ester, phenyl thioester, p-nitrophenyl thioester, p-cresyl thioester, carboxymethyl thioester, pyranyl ester, pyridyl ester, piperidyl ester, 8-quinolyl thioester, etc.], or an ester with a N-hydroxy compound [e.g. N,N-dimethylhydroxylamine, 1-hydroxy-2-(1H)-pyridone, N-hydroxysuccinimide, N-hydroxyphthalimide, 1-hydroxy-1H-benzotriazole, etc.], and the like. These reactive derivatives can optionally be selected from them according to the kind of the compound (III) to be used.

The reaction is usually carried out in a conventional solvent such as water, alcohol [e.g. methanol, ethanol, etc.], acetone, dioxane, acetonitrile, chloroform, methylene chloride, ethylene chloride, tetrahydrofuran, ethyl acetate, N,N-dimethylformamide, pyridine or any other organic solvent which does not adversely influence the reaction. These conventional solvent may also be used in a mixture with water.

In this reaction, when the compound (III) is used in a free acid form or its salt form, the reaction is preferably carried out in the presence of a conventional condensing agent such as N,N′-dicyclohexylcarbodiimide; N-cyclohexyl-N′-morpholinoethylcarbodiimide; N-cyclohexyl-N′-(4-diethylaminocyclohexyl)carbodiimide; N,N′-diethylcarbodiimide, N,N′-diisopropylcarbodiimide; N-ethyl-N′-(3-dimethylaminopropyl)carbodiimide; N,N′-carbonylbis-(2-methylimidazole); pentamethyleneketene-N-cyclohexylimine; diphenylketene-N-cyclohexylimine; ethoxyacetylene, 1-alkoxy-1-chloroethylene; trialkyl phosphite; ethyl polyphosphate; isopropyl polyphosphate; phosphorus oxychloride (phosphoryl chloride); phosphorus trichloride; diphenyl phosphorylazide; thionyl chloride; oxalyl chloride; lower alkyl haloformate [e.g. ethyl chloroformate, isopropyl chloroformate, etc.]; triphenylphosphine; 2-ethyl-7-hydroxybenzisoxazolium salt; 2-ethyl-5-(m-sulfophenyl)isoxazolium hydroxide intramolecular salt; 1-(p-chlorobenzenesulfonyloxy)-6-chloro-1H-benzotriazole; so-called vilsmeier reagent prepared by the reaction of N,N-dimethylformamide with thionyl chloride, phosgene, trichloromethyl chloroformate, phosphorus oxychloride, etc.; or the like.

The reaction may also be carried out in the presence of an inorganic or organic base such as an alkali metal bicarbonate, tri(lower)alkylamine, pyridine, N-(lower)alkylmorpholine, N,N-di(lower)alkylbenzylamine, or the like.

The reaction temperature is not critical, and the reaction is usually carried out under cooling to warming.

The compound (III) used in this process can be prepared by a conventional method.

Process 2 :

The compound (Ib) or a salt thereof can be prepared by subjecting the compound (Ia) or a salt thereof to oxidation reaction.

Suitable oxidizing agent to be used in this oxidation reaction may include conventional ones such as 2,3-dichloro-5,6-dicyano-1,4-benzoquinone (DDQ), and the like.

The present oxidation is carried out with solvent such as benzene, toluene, chloroform, dichloromethane, carbon tetrachloride, diethyl ether, dimethylformamide or any other solvent which does not adversely affect the reaction, and the solvent is optionally selected according to a kind of oxidizing agent to be used.

The reaction temperature of the oxidation reaction of this process is not critical, and the reaction is carried out under cooling, at ambient temperature, under warming or under heating. The reaction temperature is optionally selected according to a kind of oxidizing agent to be used.

Process 3 :

The compound (Id) or a salt thereof can be prepared by oxidizing the compound (Ic) or a salt thereof.

The present oxidation reaction can be carried out by a conventional metal which is applied for the

transformation of N into

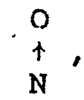

for example by using an oxidizing agent such as m-chloroperbenzoic acid, perbenzoic acid, peracetic acid, ozone, hydrogen peroxide, periodic acid or the like.

The present reaction is usually carried out in a solvent such as water, acetone, dioxane, acetonitrile, chloroform, methylene chloride, tetrahydrofuran, ethyl acetate or any other solvent which does not adversely affect the reaction.

The reaction temperature is not critical and the reaction is preferably carried out under cooling or at ambient temperature.

The object compound (I) of the present invention can be isolated and purified in a conventional manner, for example, extraction, precipitation, fractional crystallization, recrystallization, chromatography, and the like.

The object compound (I) thus obtained can be converted to its salt by a conventional method.

The object compound (I) of the present invention are novel and exhibit pharmacological activities such as 5-HT antagonism, especially, 5-$HT_3$ antagonism, and the like and therefore are useful as 5-HT antagonist for treating or preventing central nervous system (CNS) disorders such as psychosis (e.g. schizophrenia, mania, etc.), anxiety, and depression; pains or aches such as headaches (e.g. migraine, cluster headaches, vascular headaches, etc.), and neuralgia (e.g. trigeminal neuralgia, etc.); gastrointestinal disorders such as symptoms of gastrointestinal dysfunction such as occur with, for example, dyspepsia, peptic ulcer, reflux oesophagitis and flatulence, and irritable bowel syndrome (IBS); nausea or vomiting, each of which may be associated with cancer therapy; motion sickness; and the like.

Further, it is expected that the object compound (I) of the present invention are useful as therapeutical and/or preventive agents for obesity; lung embolism; arrhythmia; withdrawal syndrome resulting from addition to a drug or substance of abuse; stress-related psychiatric disorders; rhinitis; and serotonin-induced nasal disorders, and the like.

In order to illustrate the usefulness of the object compounds (I), pharmacological activities of representative compound of the present invention are shown below.

[1] Test Compound

3-Methyl-1-[(8-methyl-8-azabicyclo[3.2.1]oct-3-yl)acetyl]indole hydrochloride

[2] Test

(A) Inhibition of Benzold-Jarisch reflex

Test Method :

Male Sprague-Dawley rats weighing 260-350 g were anesthetized intraperitoneally with 1.25 g/kg urethane.

Blood pressure and heart rate were monitored continuously from the left common carotid artery with a pressure transducer. A right femoral vein was connulated for the intravenous injection (iv) of drugs. The trachea was also connulated to ease the respiration.

Rats were given a rapid bolus injection of 2-methyl-5-hydroxytryptamine (32 µg/kg, iv) to establish the control bradycardic response. Once the heart rate returned to base line, the rats were given the test compound (iv), followed by 5-minutes interval and another bolus injection of 2-methyl-5-hydroxytryptamine (32 ug/kg, iv).

| Test Result : | |
|---|---|
| Dose µg/kg) | Inhibition (%) |
| 3.2 | 52.9 |

(B) Inhibition of Cisplatin-induced vomiting

Test Method :

Nonfasted female beagles weighing about 10 kg were administered test compound or saline intravenously twice 10 minutes prior to and 90 minutes after Cisplatin dosing (3.2 mg/kg, iv).

Cisplatin was dissolved in 0.9% warm saline with a final concentration of 3 mg/ml and used immediately. The beagles were observed for vomiting for up to 5 hours following Cisplatin administration.

| Test Result : | |
|---|---|
| Dose (µg/kg) | Inhibition (%) |
| 100 | 95.2 |

For therapeutic administration, the object compound (I) of the present invention are used in the form of conventional pharmaceutical preparation which contains said compound as an active ingredient, in admixture with pharmaceutically acceptable carriers such as an organic or inorganic solid or liquid excipient which is suitable for oral, parenteral and external administration. The pharmaceutical preparations may be in solid form such as tablet, granule, powder, capsule, or liquid form such as solution, suspension, syrup, emulsion, lemonade and the like.

If needed, there may be included in the above preparations auxiliary substances, stabilizing agents, wetting agents and other commonly used additives such as lactose, citric acid, tartaric acid, stearic acid magnesium stearate, terra alba, sucrose, corn starch, talc, gelatin, agar, pectin, peanut oil, olive oil, cacao butter, ethylene glycol, and the like.

While the dosage of the compound (I) may vary from and also depend upon the age, conditions of the patient, a kind of diseases of conditions, a kind of the compound (I) to be applied, etc. In general amounts between 0.01 mg and about 500 mg or even more per day may be administered to a patient. An average singele dose of about 0.05 mg, 0.1 mg, 0.25 mg, 0.5 mg, 1 mg, 10 mg, 20 mg, 50 mg, 100 mg of the object compound (I) of the present invention may be used in treating diseases.

The following Preparations and Examples are given for the purpose of illustrating the present invention.

Preparation 1

To a solution of (8-methyl-8-azabicyclo[3.2.1]oct-3-yl)acetic acid (0.50 g) in N,N-dimethylformamide (10 ml) were added 1-hydroxy-1H-benzotriazole (0.44 g) and dicyclohexylcarbodiimide (0.68 g), and the mixture was stirred at ambient temperature for 1 hour. To the mixture was added 2,3-dihydro-3,3-dimethylindole (0.47 g), and was stirred at ambient temperature for 1 day. After poured into water (100 ml), the precipitates were filtered off. The solution was washed with ethyl acetate, and extracted by chloroform at pH = 13 using aqueous sodium hydroxide. The extract was dried over sodium sulfate, evaporated, and triturated in water (30 ml) to give crude 2,3-dihydro-3,3-dimethyl-1-[(8-methyl-8-azabicyclo[3.2.1]oct-3-yl)acetyl]indole, which was recrystallized from ethanol-water (2:8 V/V to give pure one (0.58 g).

mp : 80-83 °C

IR (Nujol) : 1640, 1595 $cm^{-1}$

NMR ($CDCl_3$, δ) : 1.33 (6H, s), 1.3-2.8 (11H, m), 2.26 (3H, s), 2.9-3.3 (2H, m), 3.75 (2H, s), 6.8-7.3 (3H, m), 8.18 (1H, br d, J=8Hz)
Mass : m/z =312 ($M^+$)

Preparation 2

The following compound was obtained according to a similar manner to that of Preparation 1.
2,3-Dihydro-3-methyl-1-[(8-methyl-8-azabicyclo[3.2.1]oct-3-yl)acetyl]indole.
mp : 76-80° C
IR (Nujol) : 1645, 1593 $cm^{-1}$
NMR ($CDCl_3$, δ) : 1.0-2.9 (17H, m), 2.9-3.25 (2H, m), 3.3-3.7 (2H, m), 4.0-4.4 (1H, m), 6.8-7.4 (3H, m), 8.20 (1H, d, J=8Hz)
Mass : m/z = 298 ($M^+$)

Example 1

To a solution of 2,3-dihydro-3-methyl-1-[(8-methyl-8-azabicyclo[3.2.1]oct-3-yl)acetyl]indole (0.7 g) in dichloromethane (10 ml) was added 2,3-dichloro-5,6-dicyclo-1,4-benzoquinone (DDQ) (0.64 g), and the mixture was stirred under reflux for 8 hours. After being cooled, the solution was washed with 1N sodium hydroxide solution and brine, dried over sodium sulfate and then evaporated. The residue was chromatographed on silica gel using chloroform-methanol (gradient 0-15% V/V) as an eluent. Removal of the solvent gave 3-methyl-1-[(8-methyl-8-azabicyclo[3.2.1]oct-3-yl)acetyl]indole (0.35 g).
IR (Neat) : 1685, 1600 $cm^{-1}$
NMR ($CDCl_3$, δ) : 1.3-2.8 (8H, m), 2.30 (3H, s), 2.35 (3H, s), 2.8-3.0 (4H, m), 3.73 (1H, br s), 7.1-7.6 (4H, m), 8.10 (1H, d, J=8Hz)

Example 2

To a solution of 3-methyl-1-[(8-methyl-8-azabicyclo[3.2.1]oct-3-yl)acetyl]indole (0.35 g) in ethanol (10 ml) was added conc. hydrochloric acid (0.12 ml). The mixture was evaporated, and the residue was triturated with isopropyl alcohol to give 3-methyl-1-[(8-methyl-8-azabicyclo[3.2.1]oct-3-yl)acetyl]indole hydrochloride (0.101 g).
mp : 180-182° C
IR (Nujol) : 1690, 1600 $cm^{-1}$
NMR (DMSO-$d_6$, δ) : 1.5-2.8 (12H, m), 3.1-3.4 (3H, m), 3.5-3.8 (3H, m), 4.31 (1H, d, J=4Hz), 7.2-7.4 (2H, m), 7.5-7.6 (1H, m), 7.78 (1H, s), 8.2-8.4 (1H, m)
Mass : m/z = 296 ($M^+$, free)

Example 3

A mixture of (8-methyl-8-azabicyclo[3.2.1]oct-3-yl)acetic acid (605 mg), 1-hydroxy-1H-benzotriazole hydrate (505 mg), and dicyclohexylcarbodiimide (681 mg) in N,N-dimethylformamide (7 ml) was stirred at room temperature for 1.5 hours. 2′,3′-Dihydrospiro[cyclopropane-1,3′-indole] (436 mg) was added and the mixture was stirred at room temperature for 40 hours. The reaction mixture was diluted with chilled water, made basic with 3N aqueous sodium hydroxide solution, and extracted with methylene chloride three times. After filtration of the insoluble material, the organic layer was washed with water twice and brine, dried over anhydrous magnesium sulfate, and evaporated in vacuo. The residue was purified by silica gel column chromatography (10% methanol chloroform) to give an oil (0.652 g). The oil was treated with a solution of maleic acid (0.244 g) in hot methanol. Crystallization from methanol-ether gave 2′,3′-dihydro-1′-[(8-methyl-8-azabicyclo[3.2.1]oct-3-yl)acetyl]spiro[cyclopropane-1,3′-indole] maleate (0.61 g).
mp : 178-180° C
IR (Nujol) : 2540, 1700, 1650, 1575, 1350 $cm^{-1}$
NMR (DMSO-$d_6$, δ) : 1.00-1.90 (4H, m), 1.72 (2H, br d, J=14Hz), 2.04-2.30 (7H, m), 2.68 (3H, s), 2.70 (2H, m), 3.81 (2H, s), 4.13 (2H, s), 6.05 (2H, s), 6.78 (1H, d, J=7Hz), 6.96 (1H, t, J=7Hz), 7.11 (1H, t, J=7Hz),

8.08 (1H, d, J=7Hz)

Example 4

The following compound was obtained according to a similar manner to that of Example 3.
2′,3′-Dihydro-1′-[(8-methyl-8-azabicyclo[3.2.1]oct-3-yl)acetyl]spiro[cyclopentane-1,3′-indole] maleate.
mp : 74-80 °C
NMR (DMSO-$d_6$, δ) : 1.70-1.85 (10H, m), 2.07-2.45 (7H, m), 2.68 (3H, s), 2.75 (2H, m), 3.81 (2H, s), 3.91 (2H, s), 6.04 (2H, s), 7.02 (1H, t, J=7Hz), 7.16 (1H, t, J=7Hz), 7.24 (1H, d, J=7Hz), 8.07 (1H, d, J=7Hz)

Example 5

A mixture of 2,3-dihydro-3,3-dimethyl-1-[(8-methyl-8-azabicyclo[3.2.1]oct-3-yl)acetyl]indole (1.1 g) and m-chloroperbenzoic acid (911 mg) in chloroform was stirred at room temperature for 2 hours. The reaction mixture was evaporated in vacuo and the residue was diluted with a mixture of ethyl acetate (20 ml ) and tetrahydrofuran (10 ml). The resultant solution was washed with aqueous sodium bicarbonate solution and brine, dried over anhydrous magnesium sulfate, and evaporated in vacuo. The residue was purified by silica gel column chromatography (10% methanol-chloroform) to give amorphous powder. The powder was dissolved with water (10 ml), 6N-hydrochloric acid (5 ml) and ether (10 ml). The mixture was stirred at ambient temperature for 1 hour. The resulting precipitates were collected to give 3-(2,3-dihydro-3,3-dimethylindol-1-yl)carbonylmethyl-8-methyl-8-azabicyclo[3.2.1]octane 8-oxide hydrochloride (0.41 g).
mp : 221-231 °C (dec.)
IR (Nujol) : 1650, 1590, 1540 $cm^{-1}$
NMR (DMSO-$d_6$, δ) : 1.31 (6H, s), 1.7-2.8 (11H, m), 3.56 (3H, s), 3.88 (2H, s), 4.17 (2H, s), 6.9-7.2 (2H, m), 7.25 (1H, d, J=7.13Hz), 8.06 (1H, d, J=7.13Hz)
Mass : m/z = 328 ($M^+$)

**Claims**

1. A compound of the formula :

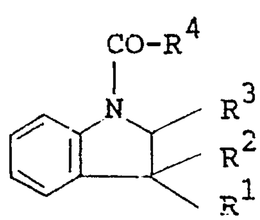

wherein
$R^1$ is lower alkyl and
$R^2$ and $R^3$ are each hydrogen, lower alkyl or linked together to form a bond; or
$R^1$ and $R^2$ are linked together to form lower alkylene and
$R^3$ is hydrogen, and
$R^4$ is azabicyclo($C_5$-$C_{12}$)alkyl(lower)alkyl or its N-oxide, each of which may be substituted by lower alkyl, provided that $R^4$ is N-oxide of azabicyclo($C_5$-$C_{12}$)alkyl(lower)alkyl, which may be substituted by lower alkyl when $R^1$ is lower alkyl and $R^2$ and $R^3$ are each hydrogen or lower alkyl,
and pharmaceutically acceptable salt thereof.

2. A process for preparing a compound of the formula :

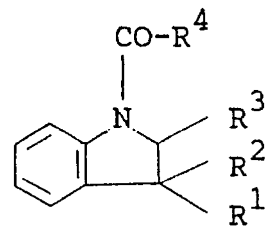

wherein
$R^1$ is lower alkyl and
$R^2$ and $R^3$ are each hydrogen, lower alkyl or linked together to form a bond; or
$R^1$ and $R^2$ are linked together to form lower alkylene and
$R^3$ is hydrogen, and
$R^4$ is azabicyclo($C_5$-$C_{12}$)alkyl(lower)alkyl or its N-oxide, each of which may be substituted by lower alkyl, provided that $R^4$ is N-oxide of azabicyclo($C_5$-$C_{12}$)alkyl(lower)alkyl, which may be substituted by lower alkyl when $R^1$ is lower alkyl and $R^2$ and $R^3$ are each hydrogen or lower alkyl,
or a salt thereof which comprises

(1) reacting a compound of the formula :

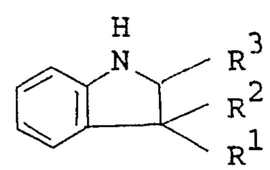

wherein $R^1$, $R^2$ and $R^3$ are each as defined above,
or its reactive derivative at the imino group or a salt thereof, with a compound of the formula :
$HOOC-R^4$
wherein $R^4$ is as defined above,
or its reactive derivative at the carboxy group or a salt thereof, to give a compound of the formula :

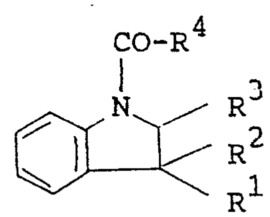

wherein $R^1$, $R^2$, $R^3$ and $R^4$ are each as defined above,
or a salt thereof, or

(2) subjecting a compound of the formula :

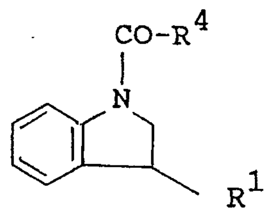

wherein $R^1$ and $R^4$ are each as defined above,
or a salt thereof, to oxidation, to give a compound of the formula :

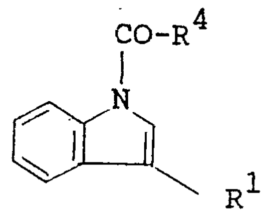

wherein $R^1$ and $R^4$ are each as defined above, or a salt thereof, or

(3) subjecting a compound of the formula :

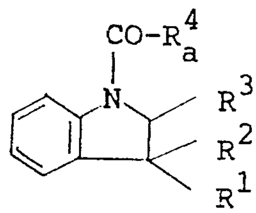

wherein

$R^1$, $R^2$ and $R^3$ are each as defined above, and

$R_a^4$ is azabicyclo($C_5$-$C_{12}$)alkyl(lower)alkyl, which may be substituted by lower alkyl, or a salt thereof, to N-oxidation, to give a compound of the formula :

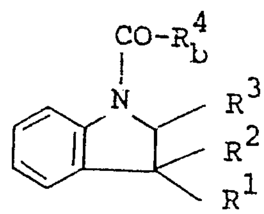

wherein

$R^1$, $R^2$ and $R^3$ are each as defined above, and

$R_b^4$ is N-oxide of azabicyclo($C_5$-$C_{12}$)alkyl(lower)alkyl, which may be substituted by lower alkyl, or a salt thereof.

3. A pharmaceutical composition which comprises a compound of claim 1 and a pharmaceutically acceptable carrier or excipient.

4. A process for preparing a pharmaceutical composition which comprises admixing a compound of claim 1 with a pharmaceutically acceptable carrier or excipient.

5. A use of a compound of claim 1 as a medicament.

6. A use of a compound of claim 1 as 5-hydroxytryptamine antagonist.

7. A use of a compound of claim 1 for manufacturing a medicament for treating 5-hydroxytryptamine mediated diseases.

8. A method for treating 5-hydroxytryptamine mediated diseases which comprises administering a compound of claim 1 to human or animals.